# EUROPÄISCHE PATENTSCHRIFT

(11) **EP 1 081 191 B1**
(45) Veröffentlichungstag und Bekanntmachung des Hinweises auf die Patenterteilung: **02.04.2003**
(21) Anmeldenummer: 00117586.8
(22) Anmeldetag: 16.08.2000
(51) Int. Cl.: C08L 83/04, A61K 6/10

(54) **Bei Raumtemperatur aushärtende Silicon-Masse II und ihre Verwendung**
Two-component at room temperature crosslinkable polysiloxane compositions and their use
Compositions de polysiloxane à deux composants durcissables à température ambiante et leur utilisation

(30) Priorität: 06.09.1999 DE 19942467
(43) Veröffentlichungstag der Anmeldung: 07.03.2001
(73) Patentinhaber: Heraeus Kulzer GmbH & Co.KG, 63450 Hanau (DE)
(72) Erfinder: Freckmann, Michael, 50769 Köln (DE); Nehren, Klaus-Dieter, 41539 Dormagen (DE); Schaub, Matthias, Dr., 40593 Düsseldorf (DE)
(74) Vertreter: Kühn, Hans-Christian

(56) Entgegenhaltungen:
- EP-A- 0 939 107
- DE-A- 3 636 974

## Beschreibung

Die Erfindung betrifft bei Raumtemperatur durch Kondensation aushärtende Silicon-Massen aus Hydroxy-Gruppen aufweisendes Polyorganosiloxan und Füllstoff enthaltender Basis-Paste sowie Vemetzer und Katalysator aus Organometallverbindung enthaltender Aktivator-Komponente.

Je nach Art der Vernetzung werden durch Kondensation vemetzende und durch Addition vernetzende Sillcon-Abformmassen unterschieden (R. Marxkors /H. Meiners, Taschenbuch der zahnärztlichen Werkstoffkunde, München Wien: Carl Hanser Verlag, 1978; Ullmann's Encyclopedia of Industrial Chemistry, 5. Auflage, Weinheim; New York: VCH, Volume 8, 1987, 288).

Die in Form eines Zwei-Komponentensystems vorliegenden Silicon-Abformmassen bestehen - wenn sie zu den durch Kondensation vernetzenden gehören - aus Polydimethylsilanole oder andere Hydroxy-Polyorganosiloxane und Fültstoff enthaltender Basis-Paste sowie Vemetzer und Katalysator für die Polykondensation enthaltender Aktivator-Flüssigkeit oder -Paste. Nach dem kurz vor Gebrauch zu erfolgenden Vermischen der beiden Komponenten reagieren die Polydimethylsilanole mit dem üblicherweise aus Kieselsäureestem oder anderen Alkoxysilanen bestehenden Vemetzer durch Kondensation unter Kettenverlängerung, -verzweigung und -vernetzung, und es bilden sich für die Abformung sehr gut geeignete gummielastische Materialien.

Durch Kondensation vernetzende Silicon-Abdruckmassen sind zum Beispiel aus EP 939 107 A2, DE 1 153 169 B1, DE 26 44 193 A1, DE 34 06 233 A1, DE 36 36 974 A1 und DE 43 32 037 A1 bekannt.

Aus EP 939 107 A2 ist eine 2-Komponenten-Silicon-Masse bekannt mit einer Aktivatorkomponente, die einen Polyether mit mindestens 2 Alkoxysilygruppen enthält

DE 1 153 169 B1 bezieht sich auf ein Verfahren zur Herstellung von elastomeren Formteilen aus zwei getrennt vorliegenden, pastenartigen Massen, die vor dem Aushärten bei Raumtemperatur miteinander gemischt werden. Die eine der Massen enthält hydroxylendblockiertes Diorganopolysiloxan und Vernetzer, zum Beispiel Kieselsäureester oder Organowasserstoffpolysiloxane, die andere durch Triorganasiloxy-Gruppen endbtockiertes Diorganopolysiloxan und den Kondensationskatalysator, zum Beispiel Dibutylzinndilacetat. Die Massen werden vor allem als Abdruck- oder Abdichtmassen für technische, künstlerische oder besonders für dentale Zwecke angewendet. Nachteilig ist, dass die das hydroxylendblockierte Diorganopolysiloxan und den Vemetzer enthaltende Masse in ihrer Wirkung bei Lagerung merklich nachlässt.

Aus DE 26 44193 A1 sind pastöse Massen für bei Raumtemperatur vulkanisierbare Polyorganosiloxane bekannt, die neben vemetzenden Substanzen und Katalysatoren (Härtungskatalysatoren) als Verdickungsmittel 3 - 40 Gewichts-% aktive hydrophile Kieselsäure und gegebenenfalls bis zu 40 Gewichts-% inaktive Füllstoffe, zum Beispiel Quarzmehl oder Titandioxid, enthalten. Vemetzende Substanzen sind Ester von Kiesel- und Polykieselsäuren, Alkylalkoxy-, Arylalkoxy- oder Alkylalkanoyloxysilane. Ihre Menge beträgt 0,1 - 10 Gewichts-Teile, bezogen auf das Polyorganosiloxan. Katalysatoren sind carbonsaure Metallsalze, wie Dibutylzinndilaurat, Zinn(II)-octanoat, Bleilaurat, Kobaltnaphthenat und Tetraisopropyltitanat, oder Amine beziehungsweise Aminsalze, wie Hexylamin, Cyclohexylamln und Butylammonlumacetat. Sie werden in einer Menge zwischen 0,1 und 10 %, bezogen auf das Polyorganosiloxan, eingesetzt. Die pastösen Massen sind in feuchtigkeitsdichten Verpackungen lagerstabil und können ebenso wie die Polyorganosiloxan-Pasten in Tuben mit ausgewähltem Öffnungsdurchmesser gefüllt und Ober die Länge der aus den Tuben gepressten Stränge dosiert werden. Die Durchmesser der Tubenöffnungen werden so gewählt, dass auf 100 Gewichts-Teile der Polyorganosiloxan-Pasten zwischen 3 und 40 Gewichts-Teile der pastösen Massen entfallen.

In DE 34 06 233 A1 werden für bei Raumtemperatur durch Kondensation oder Addition aushärtende pastenförmige Silicon-Massen bestimmte feinteilige anorganische Füllstoffe beschrieben, deren Teilchen mit Paraffinöl überzogen sind, eine mittlere Teilchengröße zwischen 1 und 25 Mikrometer aufweisen und aus Calciumcarbonat, Cristobalit oder Quarzmehl bestehen können. Die Silicon-Massen enthalten 30 - 90 Gewichts-% der Füllstoffe und werden bevorzugt in Abformmaterialien für dentale Zwecke verwendet, wobei kondensations- und additionsvemetzende Systeme unterschieden werden. Im ersten Fall enthalten die flüssigen oder pastenförmigen Aktivator-Komponenten ein carbonsaures Metallsalz und einen Kieselsäureester und die silicon-Massen Polyorganosiloxane mit zwei oder mehr Hydroxy-Gruppen im Molekül.

In DE 43 32 037 A1 wird ein kondensationsvernetzendes Silicon zur Abformung in der Zahnmedizin vorgeschlagen, dessen Haupt- und Nebenkomponenten im Verhältnis 1 : 1 angemischt und im Volumenverhältnis 1 : 1 in Kammem von Doppelkartuschen abgefüllt werden können. Die Hauptkomponente besteht aus Hydroxypolysiloxan, pyrogener Kieselsäure, Calciumcarbonat, Wasser und Dibutylzinndilaurat, die Nebenkomponente aus Cristobalit, Siliconöl und Paraffinöl. Dieses Silicon enthält jedoch keinen Vernetzer und härtet daher nicht zu einem gummielastischen Material aus.

Während bei additionsvernetzenden Silicon-Abformmassen Zwei-Komponentensysteme in Form lagerfähiger Pasten, die über das Gewicht oder das Volumen vorzugsweise im Verhältnis 1:1 dosiert werden können, bekannt sind (siehe zum Beispiel EP 0 219 660 B1), fehlen in dieser Art zu dosierende und lagerfähige Vernetzer-haltige Systeme bei den kondensationsvernetzenden Silicon-Abformmassen.

Der Erfindung liegt daher das Problem zugrunde, eine bei Raumtemperatur durch Kondensation aushärtende, aus den beiden Komponenten Basis-Paste und Aktivator Komponente bestehende Silicon-Masse der eingangs charakterisierten Art zu finden, deren beide Komponenten lagerstabil sind, im gewünschten Mischungsverhältnis zueinander dosiert und in jedern Verhältnis homogen miteinander gemischt werden können. Die Aktivator-Komponente soll eine dünnflüssige bis pastenförmige Konsistenz besitzen und die Darbietung der Silicon-Masse in Tuben, in Schlauchbeuteln, die für die gemeinsame Verwendung mit Kartuschen bestimmt und beispielsweise aus DE 296 02 111 U1 bekannt sind, und vorzugsweise in Doppelkartuschen ermöglichen. Die Silicon-Masse soll sich besonders zur Verwendung als dentale Abformmasse eignen.

Doppelkartuschen sind Zweikammer-Vorrichtungen der zum Beispiel in EP 0 378 806 B1 beschriebenen Art zum Mischen miteinander reagierender Komponenten und Ausbringen der erhaltenen pastösen Mischungen. Vor Gebrauch wird der die Doppelkartuschen ursprünglich verschließende Stopfen entfernt und ein an seinem vorderen Ende mit einer Ausbringöffnung versehener statischer Mischer eingesetzt. Mit Doppelkartuschen lassen sich automatisch das richtige Mischungsverhältnis der zu mischenden Komponenten und die Homogenität der gemischten Pasten auf einfache Weise erreichen.

Die die Lösung des Problems darstellende Silicon-Masse ist erfindungsgemäß durch folgendes gekennzeichnet:

Die Aktivatorkomponente enthält zusätzlich einen Polyether und/oder ein auf Polyether basierendes Polyadditionsprodukt, wobei der Polyether und/oder das auf Polyether basierende Polyadditionsprodukt alkoxysilylgruppenfrel ist.

Unter Polyadditionsprodukten werden im Sinne der Erfindung die als Folge einer Polyaddition gewonnenen Produkte verstanden. Die Polyaddition ist eine Polyreaktion, bei der durch vielfach wiederholte Addition von bis- oder polyfunktionellen Edukten oder Monomeren Polymere aufgebaut werden (Römpp Chemie Lexikon, 9. Auflage, Stuttgart; New York: Georg Thieme Verlag, Band 5, 3508).

Darüber hinaus hat sich die erfindungsgemäße Silicon-Masse besonders bewährt, wenn es sich bei den in der Aktivatorkomponente enthaltenden Polyethern um Homopolymere, Blockcopolymere oder statistische Copolymere der Struktur

R-[O-(CH₂)ₚ]ₘ-[O-(C_{q}H_{2q})]ₙ-OR

mit
p,q = 1-4 (unabhängig voneinander)
R = H, CₓH_{2x+1;} x = 1-100, Phenyl, Benzyl, Benzoyl
m,n beliebig, so dass Mₙ = 200-20.000,
handelt.

Polyadditionsprodukte sind zum Beispiel aus DE 36 36 974 A1 bekannt. Nach DE 36 36 974 A1 werden die dort beschrieben Ether-, Urethan- und Harnstoff-Gruppen enthaltenden Polyadditonsprodukte mit Alkoxysilyl-Gruppen einer überwiegenden linearen Molekülstruktur mit ausschließlich (cyclo) aliphatisch gebundenen Ether-, Urethan- und Hamstoffsegmenten und einem mittleren Molekulargewicht von 800 - 20000 besonders in Form von Pasten zur Herstellung genauer Abformungen bezahnter, teilbezahnter und unbezahnter Kiefer und von Gipsmodellen eingesetzt. Für diesen Zweck werden Mischungen der Polyadditionsprodukte mit einem Vemetzungsmittel, Insbesondere Tetraethoxysilan, zubereitet, die dann zur Herstellung von bei Raumtemperatur vernetzenden Abform- und Dubliermassen mit weiteren Komponenten versetzt werden.

Überraschenderweise wird durch die Mitverwendung der aus DE 36 36 974 A1 bekannten Polyadditionsprodukte in der üblicherweise aus Vemetzer und katalytisch wirksamer Organometallverbindung gebildeten Aktivator-Komponente eine aus Basis-Paste und Aktivator-Komponente bestehende Silicon-Masse geschaffen, die sich durch die sehr gute Hydrolysebeständigkeit und Lagerstabilität der Aktivator-Komponente auszeichnet. Die Konsistenz der Aktivator-Komponente lässt sich je nach Bedarf von dünnfließend bis pastenförmig einstellen. Basis-Paste und Aktivator-Komponente lassen sich sowohl manuell als auch automatisch genau dosieren und können in jedem Verhältnis homogen miteinander gemischt werden, so dass die erfindungsgemäße Silicon-Masse vorteilhaft auch In Verpackungen, die automatisches Abmessen. Dosieren und Mischen erlauben, dargeboten werden kann.

Für die erfindungsgemäßen Silicon-Massen hat sich eine Aktivator-Komponente als besonders geeignet erwiesen, die 10 - 90 Gewichts-%, vorzugsweise 30 - 60 Gewichts-%, des Polyadditionsproduktes und/oder Polyethers enthält. Die Menge an Polyadditionsprodukt richtet sich nach der gewünschten Konsistenz der Aktivator-Komponente, die je nach Bedarf dünnfließend bis pastenförmig sein kann. Die gewünschte Konsistenz ergibt sich aus der den praktischen Erfordernissen angepassten Darbietungsform der Silicon-Masse.

Vernetzer und Katalysator bilden die weiteren Bestandteile der Aktivator-Komponente.

Vorteilhafterweise enthält die Aktivatorkomponente mindestens ein metallorganisches Oxid und/oder mindestens ein Carboxylat aus der Gruppe der Metalle Sn, Zn, Fe, Pb, Ti, Zr und Co enthält.

Geeignete Katalysatoren sind die für diesen Zweck an sich bekannten Organozinn-, -titan- und -zirkoniumverbindungen, besonders Dibutyl- und Dioctylzinnoxid und Dibutylzinndilaurat.

Die Mitverwendung anorganischer Füllstoffe, wie zum Beispiel von Kieselsäure, in derAktivator-Komponente, ist zwar möglich, aber nicht unbedingt erforderlich.

Die Zusammensetzung der Basis-Paste weist keine Besonderheiten auf und entspricht der bekannter Sillcon-Massen des kondensationsvemetzenden Typs. Neben dem Hydroxy-Gruppen aufweisenden Polyorganosiloxan enthält die Basis-Paste weiterhin an sich bekannte Füllstoffe, zum Beispiel Quarz, Cristobalft, Calciumcarbonat, Natriumsilicat, Calciumsilicat und/oder Glas in üblicher Menge und Teilchengröße, und gegebenenfalls Verarbeitungshilfsmittet, wie hydriertes Rizinusöl. Ein Füllstoff-Gehalt von 5 - 50 Gewichts-% hat sich besonders bewährt.

Die erfindungsgemäße Silicon-Masse eignet sich für den Formenbau, für Einbettungen und BeSchichtungen und für ähnliche Anwendungen. Als besonders vorteilhaft erweist sich ihr Einsatz als dentale Abformmasse.

Zur näheren Erläuterung werden im folgenden für die erfindungsgemäßen Silicon-Massen geeignete Beispiele einer Basis-Paste und einer Aktivator-Komponente beschrieben.

### Beispiel 1: Basis-Komponente

In einem Vakuum-Planetenmischer werden - in der angegebenen Reihenfolge- 74 Gewichts-% Hydroxy-Gruppen aufweisendes Polydimethylsiloxan mit einer Viskosität von 2.000 mPas bei 23 °C, 25 Gewichts-% eines Gemisches anorganlscher Füllstoffe und 1 Gewichts-% anorganischer Farbstoff bei Raumtemperatur und normalem Druck mit 50 U/min 30 Minuten lang miteinander zu einer Paste vermischt. Anschließend wird die Paste noch 5 Minuten lang im Vakuum entgast Die fertige Paste wird dann a.) in Tuben, b.) in Schlauchbeutel und c.) in jeweils eine der beiden Kammem von Doppelkartuschen abgefüllt.

### Beispiel 2: Aktivator-Komponente B

Nach dem in DE 36 36 974 A1 beschriebenen Verfahren wird aus einem aus Propylenoxid, Ethylenoxid und Propylenglykol zubereiteten Polyether (87,5 Gew.-%), Isophorondiisocyanat (9,7 Gew.-%) und Hexylamin (2,8 Gew.-%) ein Polyadditionsprodukt synthetisiert, das keine endständigen Alkoxysilylgruppen enthält. 75 Gew.-% des Polyadditionsproduktes werden mit 25 Gewichts-% eines aus Vinyltrimethoxysilan und Dibutylzinnoxid durch einstundiges Erhitzen auf 120 °C unter Rückfluss und anschließendes Abkühlen lassen gewonnenen Präparates homogen miteinander vermischt. Die erhaltene Aktivator-Komponente wird a.) in Tuben, b.) in Schlauchbeutel und c.) in die jeweils 2. Kammem einer Doppeikartusche, deren erste Kammer bereits die Basis-Paste enthalten, gefüllt.

### Beispiel 3: Aktivator-Komponente D

75 Gew.-% eines Polytetrahydrofurans mit einem mittleren Molekulargewichts von 2.000 werden auf 60 °C erwärmt und mit 25 Gew.-% eines aus Vinyltrimethoxysilan und Dibutylzinnoxid durch einstündiges Erhitzen auf 120 °C unter Rückfluss und anschließendes Abkühlen lassen gewonnenen Präparates homogen miteinander vermischt. Die nach Abkühlung auf Raumtemperatur erhaltene Aktivator-Komponente wird a.) in Tuben, b.) in Schlauchbeutel und c.) in die jeweils 2. Kammem der Doppelkartuschen, deren erste Kammer bereits die Basis-Paste enthalten, gefüllt.

### Beispiel 4:

Die in den Beispielen 2 und 3 beschriebenen Aktivator-Komponenten werden jeweils mit der in Beispiel 1 beschriebenen Basis-Komponente aus einer Kartusche im Volumenverhältnis 1:4 ausgedrückt und über einen statischen Mischer homogen gemischt. In allen Fällen werden bei Raumtemperatur zu Elastomeren vemetzende Massen erhalten.

## Patentansprüche

1. Bei Raumtemperatur durch Kondensation aushärtende Silicon-Masse aus Hydroxy-Gruppen aulweisendes Polyorganosiloxan und Füllstoff enthaltender Basis-Paste sowie Vemetzer und Katalysator aus Organometallverblndung enthaltender Aktivator-Komponente, **dadurch gekennzeichnet, dass** die Aktivator-Komponente zusätzlich einen Polyether und/oder ein auf Polyether basierendes Polyadditionsprodukt enthält, wobei der Polyether und/oder das auf Polyether basierende Polyadditionsprodukt alkoxysilylgruppenfrei ist.

2. Silicon-Masse nach Anspruch 1, **dadurch gekennzeichnet, dass** es sich bei den in der Aktivatorkomponente enthaltenen Polyethem um Homopolymere, Blockcopolymere oder statistische Copolymere der Struktur
R-[O-(CH₂)ₚ]ₘ-[O-(C_{q}H_{2q})]ₙ-OR
mit
p,q = 1-4 (unabhängig voneinander)
R = H, CₓH_{2x+1;} x = 1-100, Phenyl, Benzyl, Benzoyl
m,n beliebig, so dass Mₙ = 200-20.000,
handelt.

3. Silicon-Masse nach Anspruch 1 oder 2, **dadurch gekennzeichnet, dass** die Menge an Polyadditionsprodukt und/oder Polyether in der Aktivatorkomponente 10 - 90 Gew.-% beträgt.

4. Silicon-Masse nach Anspruch 3, **dadurch gekennzeichnet, dass** die Menge an Polyadditionsprodukt und/oder Polyether in der Aktivatorkomponente 30 - 60 Gew.-% beträgt.

5. Silicon-Masse nach einem der Ansprüche 1 bis 4, **dadurch gekennzeichnet, dass** die Aktivatorkomponente mindestens ein metallorganisches Oxid und/oder mindestens ein Carboxylat aus der Gruppe der Metalle Sn, Zn, Fe, Pb, Ti, Zr und Co enthält.

6. Silicon-Masse nach Anspruch 5, **dadurch gekennzeichnet, dass** die Aktivatorkomponente Dibutyl- oder Dioctylzinnoxid enthält.

7. Silicon-Masse nach einem der Ansprüche 1 bis 6, **dadurch gekennzeichnet, dass** die Basispaste einen Füllstoffgehalt von 5 - 80 Gew.-% aufweist.

8. Silicon-Masse nach einem der Ansprüche 1 bis 7, **gekennzeichnet durch** die Darbietung von Basispaste und Aktivatorkomponente in Tuben.

9. Silicon-Masse nach einem der Ansprüche 1 bis 7, **gekennzeichnet durch** die Darbietung von Basispaste und Aktivatorkomponente In Schlauchbeuteln.

10. Silicon-Masse nach einem der Ansprüche 1 bis 7, **gekennzeichnet durch** die Darbietung von Basispaste und Aktivatorkomponente in Doppelkartuschen.

11. Verwendung der Silioon-Masse nach einem der Ansprüche 1 bis 10 als dentale Abformmasse.

## Claims

1. Silicone material curing at room temperature by condensation and comprising polyorganosiloxane having hydroxyl groups and filler-containing base paste and crosslinking agent and catalyst comprising an activator component containing an organometallic compound, **characterized in that** the activator component additionally contains a polyether and/or a polyether-based polyadduct, the polyether and/or the polyether-based polyadduct being free of alkoxysilyl groups.

2. Silicone material according to Claim 1, **characterized in that** the polyethers contained in the activator component are homopolymers, block copolymers or random copolymers having the structure
R- [O-(CH₂)ₚ]ₘ-[O-(C_{q}H_{2q})]ₙ-OR
where
p, q = 1-4 (independently of one another)
R = H, CₓH₂ₓ₊₁; x = 1-100, phenyl, benzyl, benzoyl m, n are as desired, so that Mₙ = 200-20,000.

3. Silicone material according to Claim 1 or 2, **characterized in that** the amount of polyadduct and/or polyether in the activator component is 10-90% by weight.

4. Silicone material according to Claim 3, **characterized in that** the amount of polyadduct and/or polyether in the activator component is 30-60% by weight.

5. Silicone material according to any of Claims 1 to 4, **characterized in that** the activator component contains at least one organometallic oxide and/or at least one carboxylate from the group consisting of the metals Sn, Zn, Fe, Pb, Ti, Zr and Co.

6. Silicone material according to Claim 5, **characterized in that** the activator component contains dibutyl- or dioctyltin oxide.

7. Silicone material according to any of Claims 1 to 6, **characterized in that** the base paste has a filler content of 5-80% by weight.

8. Silicone material according to any of Claims 1 to 7, **characterized by** the provision of base paste and activator component in tubes.

9. Silicone material according to any of Claims 1 to 7, **characterized by** the provision of base paste and activator component in tubular bags.

10. Silicone material according to any of Claims 1 to 7, **characterized by** the provision of base paste and activator component in double cartridges.

11. Use of the silicone material according to any of Claims 1 to 10 as a dental impression compound.

## Revendications

1. Masse de silicone durcissant par condensation à la température ambiante constituée par une pâte de base contenant un polyorganosiloxane comportant des groupes hydroxyle et une charge ainsi qu'un composant activateur contenant un réticulant et un catalyseur constitué par un composé organométallique, **caractérisée en ce que** le composant activateur contient en outre un polyéther et/ou un produit de polyaddition à base de polyéther, où le polyéther et/ou le produit de polyaddition à base de polyéther sont dépourvus de groupes alcoxysilyle.

2. Masse de silicone selon la revendication 1 **caractérisée en ce que** les polyéthers contenus dans le composant activateur consistent en homopolymères, copolymères séquencés ou copolymères statistiques de structure
R-[O-(CH₂)ₚ]ₘ-[O-(C_{q}H_{2q})]ₙ-OR
avec
p, q = 1-4 (indépendamment l'un de l'autre)
R = H, CₓH₂ₓ₊₁ ; x = 1-100, phényle, benzyle, benzoyle
m, n quelconques de manière que Mₙ = 200-20 000.

3. Masse de silicone selon la revendication 1 ou 2 **caractérisée en ce que** la quantité de produit de polyaddition et/ou de polyéther dans le composant activateur est 10-90 % en masse.

4. Masse de silicone selon la revendication 3 **caractérisée en ce que** la quantité de produit de polyaddition et/ou de polyéther dans le composant activateur est 30-60 % en masse.

5. Masse de silicone selon l'une des revendications 1 à 4 **caractérisée en ce que** le composant activateur contient au moins un oxyde organométallique et/ou au moins un carboxylate du groupe des métaux Sn, Zn, Fe, Pb, Ti, Zr et Co.

6. Masse de silicone selon la revendication 5 **caractérisée en ce que** le composant activateur contient de l'oxyde de dibutyl- ou dioctylétain.

7. Masse de silicone selon l'une des revendications 1 à 6 **caractérisée en ce que** la pâte de base présente une teneur en charge de 5-80 % en masse.

8. Masse de silicone selon l'une des revendications 1 à 7 **caractérisée par** la fourniture de la pâte de base et du composant activateur dans des tubes.

9. Masse de silicone selon l'une des revendications 1 à 7 **caractérisée par** la fourniture de la pâte de base et du composant activateur dans des récipients souples.

10. Masse de silicone selon l'une des revendications 1 à 7 **caractérisée par** la fourniture de la pâte de base et du composant activateur dans des cartouches doubles.

11. Utilisation de la masse de silicone selon l'une des revendications 1 à 10 comme masse de moulage dentaire.
